# EUROPEAN PATENT APPLICATION

(11) **EP 4 382 031 A1**
(43) Date of publication of application: **12.06.2024**
(21) Application number: 22306840.4
(22) Date of filing: 09.12.2022
(51) Int. Cl.: A61B 3/028, A61B 3/103, A61B 3/14, A61B 3/04

(54) **APPARATUS AND METHOD FOR DETERMINING REFRACTION ERROR OF AT LEAST AN EYE OF A SUBJECT**

(71) Applicant: Essilor International, 94220 Charenton-Le-Pont (FR)
(72) Inventor: PELOUX, Marius, 94220 Charenton le Pont (FR); PINAULT, Philippe, 94220 Charenton le Pont (FR); BOUTINON, Stéphane, 94220 Charenton-le-Pont (FR)
(74) Representative: Jacobacci Coralis Harle

(57) **Abstract**

The invention concerns an apparatus (200) for determining refraction error of at least an eye (1) of a subject, the apparatus comprising a corrective system (110) comprising a variable power optical system, a measurement system of the refraction error of said eye comprising a light source (31), an illumination optical system (33) adapted to direct the light beam towards the eye (1) of the subject so as to form a reflected light beam (34), an image detection system (35), and a beam splitter (10) adapted to direct the reflected light beam (34) towards the image detection system (35), the beam splitter (10) enabling simultaneously the user to see a target scene through the optical beam splitter (10) and the variable power optical system and a computing system comprising an image processing system adapted to process said image and deduce therefrom a measurement of the refraction error of said eye.

## Description

### TECHNICAL FIELD OF THE INVENTION

The invention relates to the field of optometry. It relates more particularly to a system for automatic measurement of eye refraction defects of an individual.

More precisely the invention relates to a system and a method of combined objective and subjective refraction measurement of at least one eye of a subject.

### BACKGROUND INFORMATION AND PRIOR ART

Numerous documents describe devices and methods for measuring objective refraction error of an eye or for estimating subjective refraction error of said eye.

In particular, a phoropter based on a set of trial lenses enables estimating subjective refraction error, in monocular or binocular vision, and at various vision distances.

An autorefractometer, based on measuring a light beam reflected on the eye, is commonly used for measuring objective refraction error of an eye.

Today subjective refraction measurement remains the reference for establishing a prescription for corrective lenses, because it allows to adjust the optical power of the trial lens and to better manage the accommodation and the binocular vision.

Nevertheless, objective refraction measurement is very helpful to accelerate the subjective refraction process with a good starting point, especially for astigmatism. However, today, the objective refraction method is not sufficient alone to prescribe lenses.

Today trial lenses are the cheapest solution to perform subjective refraction error measurement. However, a set of trial lenses remains uneasy to use.

Automatic phoropters have more and more automatic processes, but they are not transportable and are expensive.

Recently, a commercial system has been disclosed that provides a phoropter and a binocular auto-refractometer in the same device. However, such a device is expensive and very bulky.

There is a need for an optometry measurement system and method providing an easier access to eye refraction determination, at a reasonable cost, and combining objective and subjective refraction, in shorter time than previous methods and systems.

There is a need for an optometry measurement system and method that is mobile and lightweight enabling to determine objective and subjective refraction error at various vision distances.

### SUMMARY OF THE INVENTION

Therefore one object of the invention is to provide an apparatus for determining refraction error of at least an eye of a subject, the apparatus comprising:
- a corrective system comprising a variable power optical system mounted in an eyewear device adapted to be worn by the user so that the user is able to see a target scene through the variable power optical system and a device for varying the variable power of said optical system;
- a beam splitter, the variable power optical system being arranged between the eye of the subject and the beam splitter,
- a measurement system of the refraction error of said eye, the measurement system comprising a light source adapted to emit a light beam, an illumination optical system adapted to direct the light beam towards the eye of the subject by reflection on said beam splitter so as to form a reflected light beam by reflection on the retina of said eye, and an image detection system;
- the beam splitter being adapted to direct the reflected light beam towards the image detection system, the beam splitter enabling simultaneously the user to see the target scene through the optical beam splitter and the variable power optical system;
- the image detection system being adapted to detect an image of the reflected light beam by transmission through the variable power optical system;
- a computing system comprising an image processing system adapted to process said image and deduce therefrom an estimation of the refraction error of said eye, the computing system being adapted to send a feedback signal to the device for varying the variable power of said optical system, the feedback signal depending on the estimation of the refraction error of said eye.

According to a particular aspect, the image processing system is adapted to determine a two-dimension point spread function of the image of the reflected light beam and to deduce the estimation of the refraction error of said eye from the two-dimension point spread function.

According to another particular and advantageous aspect, the measurement system comprises a coded aperture, said coded aperture having an asymmetrical shape with respect to a rotation about an optical axis of said measurement system.

For example, the coded aperture comprises a portion of a disk or a two-dimensional bar code.

Advantageously, the image processing system is adapted to determine a sharpness of the image of the reflected light beam and to further deduce the estimation of the refraction error of said eye from the sharpness.

According to a particular aspect, the apparatus is adapted to enable determination of a visual performance, such as visual acuity or contrast, of the eye viewing the target scene through the corrective system and the beam splitter.

According to a second and third embodiment, the apparatus comprises another beam splitter arranged between the variable power optical system and the eye of the subject.

Advantageously, the illumination optical system is adapted to collimate the light beam on a cornea of the eye or, respectively, focus the light beam on the cornea of the eye.

According to a particular aspect, the apparatus comprises means for moving the target scene and wherein the measurement system is adapted to estimate an accommodation of the eye in response to the moving of the target scene.

Advantageously, the apparatus is adapted for binocular vision.

According to a particular aspect, the apparatus comprises an adjustment system for adjusting an interpupillary distance.

A further object of the invention is to provide a method for determining refraction error of at least an eye of a subject, the method comprising the steps of:
a) placing a variable power optical system mounted in an eyewear device adapted to be worn by the user so that the user is able to see a target scene through the variable power optical system;
b) emitting a light beam and directing the light beam towards the eye of the subject so as to form a reflected light beam by reflection on the retina of the eye;
c) directing the reflected light beam towards an image detection system by transmission through the variable power optical system and by reflection on a beam splitter, the variable power optical system being arranged between the eye of the subject and the beam splitter, the beam splitter enabling simultaneously the user to see the target scene through the optical beam splitter and the variable power optical system;
d) detecting an image of the reflected light beam formed on the image detection system;
e) processing said image to deduce therefrom an estimation of the refraction error of said eye and a feedback signal depending on the estimation,
f) applying the feedback signal for varying the variable power of said optical system;
g) iterating the steps d) to f) until an image of the target scene on the retina meets a predetermined ending criterion.

According to a particular aspect, the method further comprises a step of checking a visual performance, such as visual acuity or contrast, of said eye based on a feedback signal of the wearer viewing the target scene through the corrective system and the beam splitter.

Advantageously, the apparatus comprises a remote controller adapted for driving the device for varying the variable power of said optical system.

### DETAILED DESCRIPTION OF EXAMPLE(S)

The following description with reference to the accompanying drawings will make it clear what the invention consists of and how it can be achieved. The invention is not limited to the embodiment/s illustrated in the drawings. Accordingly, it should be understood that where features mentioned in the claims are followed by reference signs, such signs are included solely for the purpose of enhancing the intelligibility of the claims and are in no way limiting on the scope of the claims.

In the accompanying drawings:
- Figure 1 schematically represents an apparatus for determining refraction error of an eye of a subject according to the present disclosure;
- Figure 2 schematically represents an apparatus for determining refraction error of an eye of a subject according to a first embodiment of the present disclosure with an emmetropic eye;
- Figure 3 schematically represents an apparatus for determining refraction error of an eye of a subject according to the first embodiment with a myopic eye ;
- Figure 4 schematically represents different shapes of aperture for the measurement system, and the corresponding images detected as a function of the distance;
- Figure 5 schematically represents a binocular apparatus for determining refraction error of the two eyes of a subject according to the present disclosure;
- Figure 6 schematically represents an apparatus for determining refraction error of an eye of a subject according to a second embodiment;
- Figure 7 schematically represents an apparatus for determining refraction error of an eye of a subject according to a third embodiment;
- Figure 8 schematically represents an apparatus for determining refraction error of an eye of a subject remotely driven by a smartphone or a tablet.

In the description which follows the drawings are not necessary to scale and certain features may be shown in generalized or schematic form in the interest of clarity and conciseness or for informational purposes. In addition, although making and using various embodiments are discussed in detail below, it should be appreciated that as described herein are provided many inventive concepts that may be embodied in a wide variety of contexts. Embodiments discussed herein are merely representative and do not limit the scope of the invention. It will also be obvious to one skilled in the art that all the technical features that are defined relative to a process can be transposed, individually or in combination, to a device and conversely, all the technical features that are defined relative to a device can be transposed, individually or in combination, to a process.

### Device

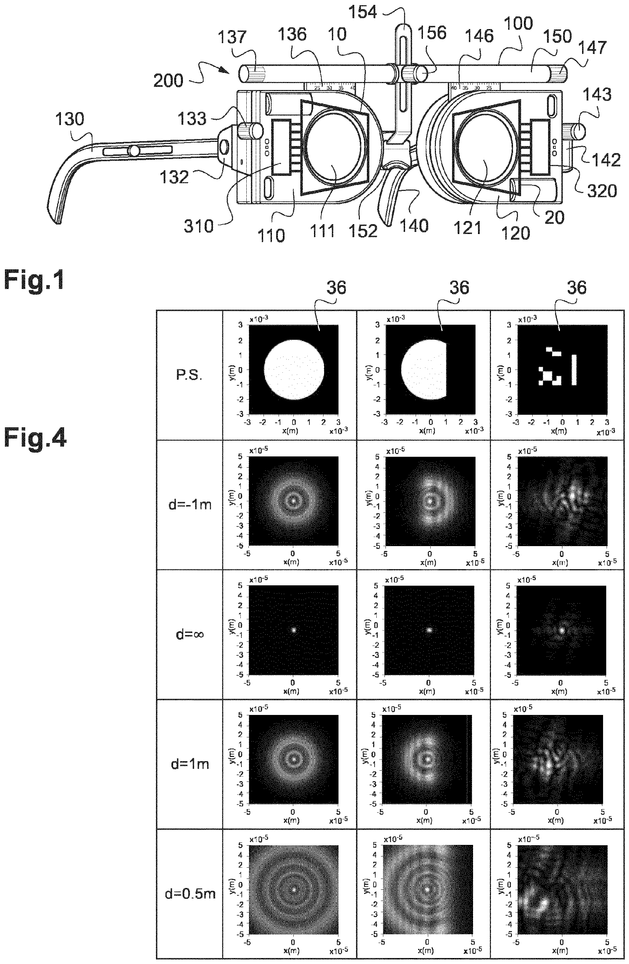

Figure 1 shows an exemplary apparatus 200 for determining refraction error of an eye of a subject according to the present disclosure. The apparatus 200 comprises an eyewear device 100 comprising a corrective system with a variable power optical system, said eyewear device 100 being adapted to be worn by a user.

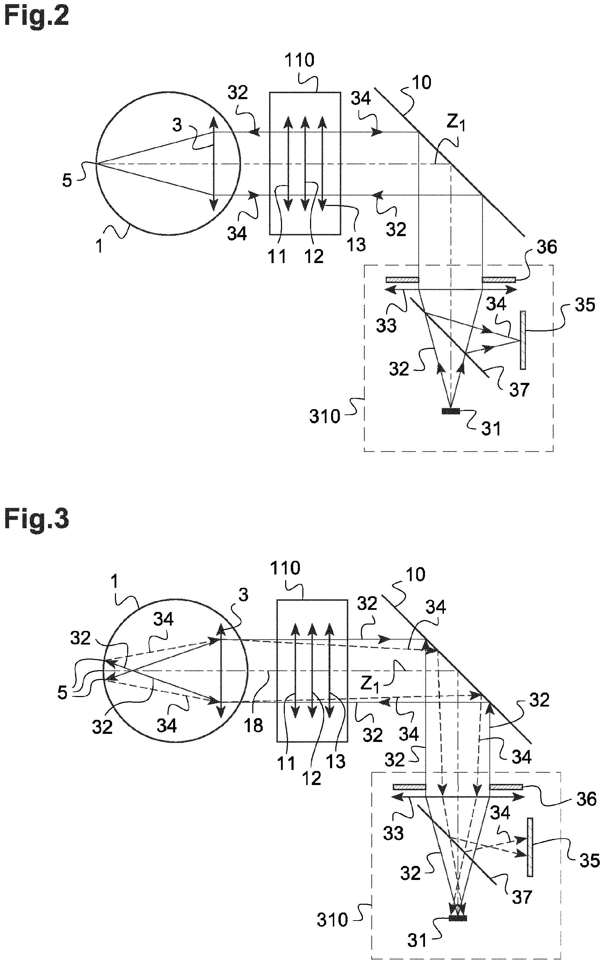

According to the present disclosure, the apparatus 200 combines the eyewear device 100 with an embedded measurement system of the refraction error 310, 320 for at least one eye, as illustrated in more details on Figures 2, 3 and 5-7.

The measurement system of the refraction error 310, 320 is a system which shines light on the retina and observes its reflection.

The eyewear device 100 comprises a vision compensating device 110 for the right eye of the subject and a vision compensating device 120 for the left eye of the subject. The vision compensating devices 110, 120 are mounted on a crosspiece 150 and equipped with branches 130, 140.

The two vision compensating devices 110, 120 are here identical but mounted on support means on a wearer's face, as explained in greater detail hereinbelow, so as to be disposed symmetrically with respect to a median vertical plane M which corresponds to the wearer's sagittal plane.

Specifically, the vision compensating device 110 is intended for the wearer's right eye. Stated otherwise, the axis Z1 of the vision compensating device 110 is parallel to the median plane M (wearer's sagittal plane).

Likewise, the vision compensating device 120 is intended for the wearer's left eye. Stated otherwise, the axis Z2 of the vision compensating device 120 is parallel to the median plane M (wearer's sagittal plane).

Each vision compensating device 110, 120 comprises a space adapted to receive optical elements of the corrective system 111, 121, also called trial lenses. The trial lenses can be, for example, placed manually.

Preferably, each vision compensating device 110, 120 comprises active digital trial lenses as described in patent document US 10,278,573. This patent document discloses optical elements (trial lenses) comprising a lens 11 of variable spherical power S_{V}, a concave planar-cylindrical lens 12, of negative cylindrical power -C₀, and a convex planar-cylindrical lens 13, of cylindrical power C₀. These optical elements 11, 12, 13 are mounted in series along an optical axis Z1, Z2 of each vision compensating device 110, 120. The optical axis is perpendicular to the cylinder axis of the first and second optical elements and the first and second optical elements do not exert any spherical power for the said direction of gaze 18 of the wearer. For example, the planar-cylindrical lenses 12, 13 have respectively a cylindrical power of -C₀ and C₀, here with C₀ = 5D (D being the dioptre, the unit for measuring vergence, inverse to the focal length expressed in metres).

The lens 11 of variable spherical power Sv is for example a lens of the liquid lens type described in patent document EP 2 034 338. Such a lens comprises a cavity closed by a transparent deformable membrane and a planar movable transparent wall; the cavity contains a transparent liquid of constant volume that is constrained, to a greater or lesser degree, by the movable face, in order to deform the membrane that is thus either a spherical concave surface, or a planar surface, or a spherical convex surface. In the lens used, a transmission made up of a nut/bolt system ensures transformation of rotary and linear motion. In the example described here, the lens 6 has a variable focal length of between -40 mm and 40 mm, i.e. a variable spherical power Sv of between -25D and +25D.

Alternatively, the variable power optical system comprises Alvarez lens doublets or rotating lenses, an active Fresnel lens based on liquid crystals, a spatial light modulator based on liquid crystals, a deformable mirror and/or a variable prism.

Advantageously, each vision compensating device 110, 120 comprises a control system and for example motors for adjusting the angular positions of the two cylindrical lenses independently of one another and for adjusting the variable spherical power of the spherical lens. Generally, a computing system comprising an image processing system is adapted to send a signal to a device for varying the variable power of said optical system. Advantageously, the image processing system is adapted to determine a sharpness of the image of the reflected light beam and to deduce the estimation of the refraction error of said eye from the sharpness of the detected image.

Still alternatively, the optical elements 111, 121 comprise conventional trial lenses placed on a plurality of discs that are rotated manually or using a motorized mechanism. In this case, the apparatus 200 comprises at least one display device adapted to display an indication of a selected lens mounted or to be mounted in each vision compensating device 110, 120 of the eyewear device 100, the selected lens being chosen from the set of trial lenses. In this case, a feedback signal is generally used to select an appropriate lens so as to reduce, for example, the blur.

The eyewear device 100, for example as illustrated on figure 1, comprises two branches 130, 140 mounted respectively on the vision compensating device 110, 120 respectively, each time on a lateral end face of the vision compensating device 110, 120 concerned and by means of a lateral fastener 132, 142. Thus, the lateral fastener 132, 142 is fixed on the lateral end wall of the vision compensating device concerned 110, 120 (that is to say, for the lateral fastener 132, on the right of the vision compensating device 110 intended for the wearer's right eye and, for the lateral fastener 142, on the left of the vision compensating device 120 intended for the wearer's left eye). Alternatively, the eyewear device 100 is a head helmet.

In the present document, the vision compensating device 110, 120 is also called a corrective system.

Each branch 130, 140 is mounted on the corresponding lateral fastener 132, 142 with a possibility of rotational adjustment about a horizontal axis (parallel to the axis Z1, Z2 defined hereinabove) for example by means of a thumbwheel 133, 143 so as to be able to tailor the pantoscopic angle.

The vision compensating devices 110, 120 are both mounted on a crosspiece 150 forming a frame element, on either side of the median plane M, respectively by means of a first slider 136 and of a second slider 146. The position of each of the first and second sliders 136, 146 is adjustable in translation along the direction of extension of the crosspiece 150 (for example by means of a thumbwheel 137, 147 provided for this purpose), thereby allowing adjustment in a horizontal direction perpendicular to the median plane M (aligned with the wearer's sagittal plane) of the position of each vision compensating device 110, 120. It is thus possible to adapt (independently of one another) the respective positions of the vision compensating devices 110, 120 to suit the half inter-pupillary distances on the wearer's right side and left side.

A nasal support 152 (designed to press on the upper portion of the wearer's nose) is mounted on the crosspiece 150, in the middle of the latter (that is to say at the level of the median plane M), by way of a central fastener 154 provided with an oblong opening which receives a peg secured to the crosspiece 150 so as to allow adjustment in a vertical direction of the relative position of the nasal support 152 and of the crosspiece 150. This adjustment is performed for example by means of a thumbwheel 156 provided for this purpose.

Provision may be made furthermore for a possible rotation of the central fastener 154 about the horizontal axis of extension of the crosspiece 150 to adjust the position of the nasal support 152 depth-wise (i.e., along the optical axis of the vision compensating devices 110, 120).

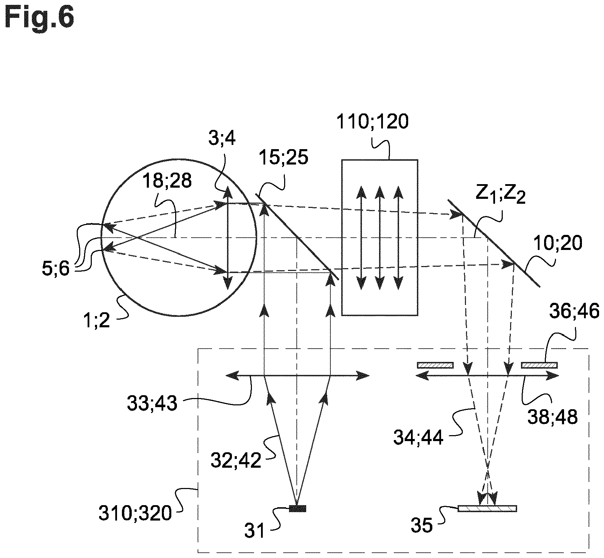

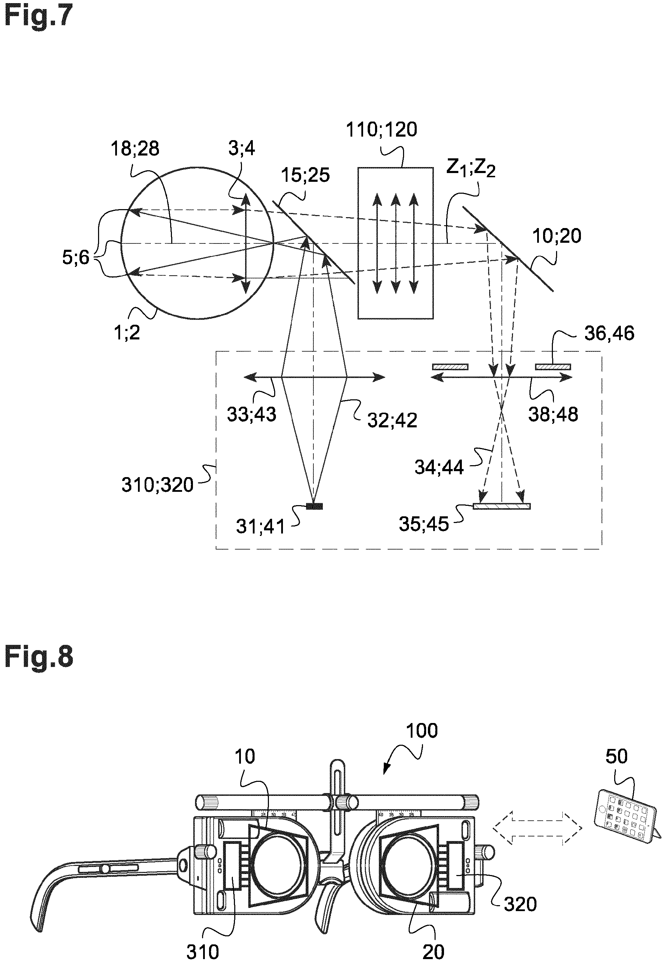

As indicated above, the eyewear device 100 is combined with an embedded measurement system of the refraction error 310, 320 for each eye, as illustrated for example on Figures 2, 3 and 5 according to a first embodiment, on figure 6 according to a second embodiment and respectively on figure 7 according to a third embodiment. To that end, the eyewear device 100 comprises a beam-splitter 10, respectively 20, arranged in front of the vision compensating device 110, respectively 120.

Figure 2 shows an apparatus for determining refraction error of at least an eye 1 of a subject according to the first embodiment. The apparatus comprises a vision compensating device 110 as detailed above, combined, via a beam-splitter 10, with a measurement system of the refraction error 310 of said eye 1 (or retinoscope). The measurement system of the refraction error 310 comprises a light source 31, a beam-splitter 37, an illumination optical system 33 having a pupil 36 and an image detection system 35. The light source 31 is for example a point light source, such as a near infrared light emitting diode or NIR LED. The measurement system of the refraction error 310 and the beam-splitter 10 are generally arranged in a casing attached to or integrated into the vision compensating device 110.

The beam-splitter 10 is arranged on the optical path between the vision compensating device 110 and the measurement system of the refraction error 310, so that the eye 1 of the wearer can see a target scene through the beam-splitter 10 and the vision compensating device 110. Advantageously, the beam splitter 10 comprises a dichroic mirror adapted for reflecting near infrared light from the light source 31, and for transmitting light in the visible range. The measurement system of the refraction error 310 is placed sideways respectively to the gaze axis 18 of the eye 1 and does not hinder the user from viewing the target scene.

The light source 31 of the measurement system of the refraction error 310 emits a light beam 32 that passes through the beam-splitter 37. The illumination optical system 33 collimates the light beam 32 that passes through the pupil 36. The beam-splitter 10 directs at least a part of the collimated light beam 32 towards the eye 1 of the subject through the vision compensating device 110. On figure 2, the eye of the subject is assumed to be an emmetropic eye watching a far distance point through the vision compensating device 110 and the beam-splitter 10. In a first step, the vision compensating device 110 is set to provide a null optical power (0D). The crystalline lens 3 of the eye 1 focuses the incident light beam 32 on a point 5 on the retina of the eye 1. The retina reflects diffusely at least a part of the light beam and forms a reflected light beam 34. The reflected light beam 34 is collimated by the crystalline lens 3 of the eye 1 and passes through the vision compensating device 110. The beam-splitter 10 directs at least a part of the reflected light beam 34 towards the measurement system of the refraction error 310. The illumination optical system 33 collects the reflected light beam 34 passing through the pupil 36. The beam-splitter 37 directs the reflected light beam 34 towards the image detection system 35.

The image detection system 35 is optically conjugated relatively to the illumination optical system 33, which means that the light source 31 and the image detection system 35 are placed at the same distance from the beam-splitter 37. If the eye 1 is perfectly emmetrope and the optical power of the vision compensating device 110 is set to 0D, the image of the reflected light beam 34 on the image detection system 35 is a point.

As illustrated on figure 3, the eye 1 presents a refractive error, for example myopia (- 2D), and the vision compensating device 110 is set to provide a null optical power (0D) in a first step. The optics of the eye 1 focuses the incident light beam 32 behind the retina and thus illuminates a surface on the retina of the eye 1 diffusing the light. The image of the reflected light beam 34 on the image detection system 35 is a spread point.

Thus, the surface and the shape of the image detected by the image detection system 35 give an estimation of the eye refraction error. The computing system comprises an image processing system adapted to process said image and deduce therefrom an estimation of the refraction error of said eye, the computing system being adapted to send a feedback signal to the device for varying the variable power of said optical system, the feedback signal depending on the estimation of the refraction error of said eye.

A practitioner and/or a control system drive the optical power (variable sphere and/or cylinder, axis) of the vision compensating device 110, to minimize the point spread function until getting a point response on the image detection system 35. This enables to determine an objective refraction measurement of the eye, while the wearer is watching a far distance point through the vision compensating device 110 and the beam-splitter 10. Then, the subjective refraction measurement is made, without removing the apparatus comprising the vision compensating device 110 and the measurement system of the refraction error 310.

The apparatus enables checking the visual performance of the eye based on a feedback signal of the wearer viewing the target scene through the corrective system and the beam splitter. The visual performance includes for example the visual acuity or the contrast of the eye.

The apparatus for determining refraction error of at least an eye of a subject enables to determine objective and subjective refraction in natural conditions at different distances, for example looking at a chart in far vision or in near vision or in intermediate vision conditions.

Combined with a moving target, for a far distance to a near distance, the apparatus for determining refraction error of the eye(s) of a subject also enables to estimate in real time the accommodation. The spread of the image on the image detection system 35 gives an estimation of the defocus produced by the accommodation, which can be compared to the need of accommodation : D = acc_meas - 1/dist_target, where D represents the difference of refraction measurement, acc_meas represents the accommodation measurement and dist_target represents the distance to the target scene, 1/dist_target is the accommodation needed. This gives an indication of the lead or lag of accommodation and the maximum of accommodation (for addition prescription). This measurement of accommodation can be done for an individual eye in monocular vision condition. In binocular vision conditions, this measurement of accommodation can be done for the two eyes provided that the movements of the eyes due to the convergence are followed.

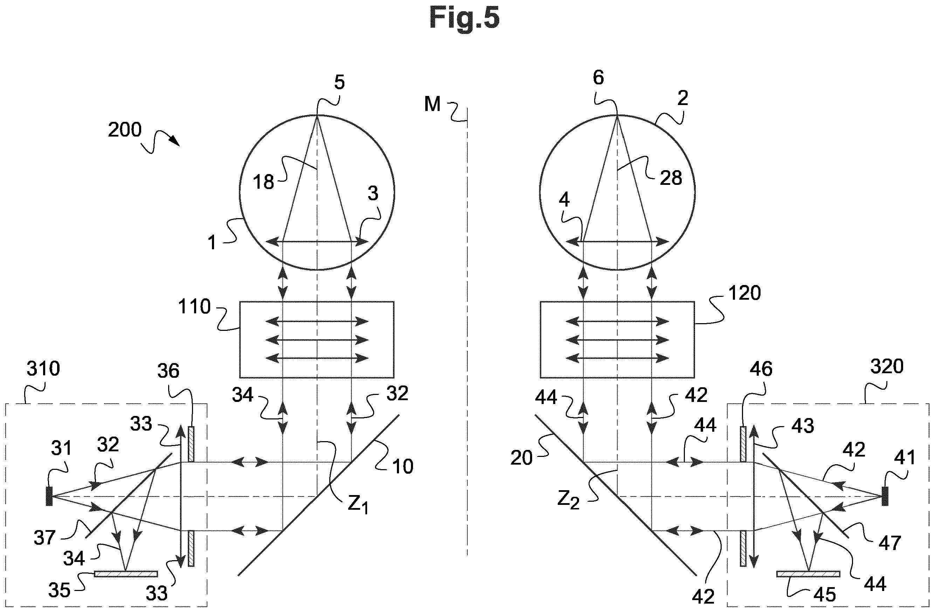

Figure 5 shows a binocular apparatus comprising a vision compensating device 110 of variable power with an embedded measurement system of the refraction error 310 for the right eye and a vision compensating device 120 of variable power with an embedded measurement system of the refraction error 320 for the left eye 2. For a binocular use, the inter-pupillary distance (IPD) is adjusted using for example the first slider 136 and the second slider 146 illustrated on figure 1. The IPD adjustment is manual or motorized. Other ergonomic adjustments are also made, like the frame height, branch length or pantoscopic angle using for example the central fastener 154, and, respectively, the lateral fasteners 132, 142 illustrated on figure 1.

The apparatus 200 for determining refraction error of the two eyes 1, 2 of a subject further comprises a vision compensating device 120 similar to the vision compensating device 110 as detailed above combined, via a beam-splitter 20, with a measurement system of the refraction error 320 of the left eye 2 (for example a retinoscope), similar to the measurement system of the refraction error 310 of the right eye 1. Likewise, the measurement system of the refraction error 320 comprises a light source 41 for example a near infrared light emitting diode or NIR LED, a beam-splitter 47, an illumination optical system 43 having a pupil 46 and an image detection system 45. The image detection system 45 is optically conjugated with the point light source 41 when the vision compensating device 120 is set to provide a null optical power (0D) and the eye 2 of the user watching a far distance point is emmetropic.

The beam-splitter 20 is arranged between the vision compensating device 120 and the measurement system of the refraction error 320, so that the eye 2 of the wearer can see a target scene through the beam-splitter 20 and the vision compensating device 120. Advantageously, the beam splitter 20 comprises a dichroic mirror adapted for reflecting near infrared light from the light source 41, and for transmitting light in the visible range. The measurement system of the refraction error 320 is placed sideways on the temple side respectively to the gaze axis 28 of the eye 2 and does not hinder the user from viewing the target scene.

The measurement system of the refraction error 320 and the beam-splitter 20 are generally arranged in a casing attached to or integrated into the vision compensating devices 120.

The light source 41 of the measurement system of the refraction error 320 emits a light beam 42 that passes through the beam-splitter 47. The illumination optical system 43 collimates the light beam 42 that passes through the pupil 46. The beam-splitter 20 directs at least a part of the collimated light beam 42 towards the eye 2 of the subject through the vision compensating device 120.

On figure 5, the eyes of the subject are assumed to be emmetropic eyes watching a far distance point through the vision compensating devices 110, 120 and the beam-splitters 10, 20. In a first step, the vision compensating devices 110, 120 are set to provide a null optical power (0D). The crystalline lens 4 of the eye 2 focuses the incident light beam 42 on a point 6 on the retina of the eye 2. The retina reflects at least a part of the light beam and forms a reflected light beam 44. The reflected light beam 44 is collimated by the crystalline lens 4 of the eye 2 and passes through the vision compensating device 120. The beam-splitter 20 directs at least a part of the reflected light beam 44 towards the measurement system of the refraction error 320. The illumination optical system 43 collects the reflected light beam 44 passing through the pupil 46. The beam-splitter 47 directs the reflected light beam 44 towards the image detection system 45.

Thus, the apparatus 200 enables simultaneous binocular refraction error measurements (objective and then subjective) of the two eyes of the subject without moving the eyewear device relatively to the user's face.

Additional options may be embedded into the apparatus for determining refraction error of at least an eye of a subject. For example, the apparatus further comprises one or two side cameras to measure the vertex distance, that is the apex of the eye to the back surface of the active lenses of the vision compensating device 110, 120. The apparatus may also comprise mechanical or electronic shutter(s) placed on the optical path of the vision compensating device 110 or 120 to enable performing monocular tests on one eye of the subject. According to another option, the apparatus comprises off-axis LEDs, arranged preferably in the same plane as the light source 31,41, to measure peripheral refraction. According to still another option, the apparatus comprises at least one eye-tracker camera to measure the pupil diameter of the eye 1, 2, which is an important parameter to optimize the calculation of the defocus value and to adjust automatically the IPD.

According to another option, the apparatus comprises other cameras for a remote clinical exam of the eye such as dry-eye condition, or to detect corneal or retinal images. According to still another option, the apparatus comprises at least one 3D time-of-flight sensor in front of the apparatus, the 3D time-of-flight sensor being adapted to measure the distance to the test such as a screen, a test chart, or a mobile target. This last option enables to obtain more accurate refraction error measurements and allows an automatic measurement of the maximum of accommodation.

According to a particular aspect of the present disclosure, the measurement system of the refraction error 310 comprises a pupil 36,46 having a coded aperture. Figure 4 is a table illustrating various pupil shapes presented on the first line and the detected images corresponding to each pupil shape, simulated at various distances d. On figure 4, we simulate, in the scope of paraxial approximation, the case where a point light source 31, 41 (at a wavelength of λ=860nm) is placed at a distance d from an optical system 33, 43 of focal length fₑ. We observe the point spread function (PSF) in 2D on the plane of the image detection system 35, 45 placed at a distance dₑ = fₑ from the optical system 33, 43. For the simulations, the distance dₑ=fₑ=1/60 meters, and the pupil radius is equal to 2mm.

More precisely, figure 4 presents, as non-limitative examples, three types of pupil shapes (P.S.) for the pupil of the measurement system of the refraction error 310, 320. The first type of pupil shape is a conventional circular or disk shape having a clear central aperture with a diameter of about 4 millimeters and centered on the optical axis of the illumination optical system 33, 43. The second type of pupil shape is a coded aperture, for example a disk shape with an opaque edge on one side of the circular pupil, the disk having a diameter of about 4 millimeters and centered on the optical axis of the illumination optical system 33, 43, and the edge having a length of about 3,5 mm. The third type of pupil shape is another coded aperture, for example a 2D bar code, comprising an arrangement of small clear geometrical shapes (squares or rectangles) on a dark background. Thus, the second and third types of pupil shape present an asymmetry with respect to a rotation of 90 degrees about the optical axis, contrary to the circular pupil shape.

Whatever the pupil shape, the PSF in 2D is minimum and appears as a point, when the distance d is infinite (or in practice higher than or equal to 4m to 6m.

We observe on figure 4, in the column corresponding to the disk-shaped pupil, that the PSF size depends on the absolute value of the distance d. However, there is no difference between the PSFs corresponding to the distance d = -1m and d = 1m for instance. This pupil shape does not give any indication as to the direction into which the optical power should be varied in the refraction measurement process.

We observe on figure 4, in the column corresponding to the pupil with a side edge (or D-shaped pupil), that the PSFs corresponding to d = -1m and d = 1m are no longer identical. This difference can be used by a dedicated algorithm to detect the direction into which the optical power of the active lens(es) should move to target faster the user's prescription.

We observe on figure 4, in the column corresponding to the pupil with a coded aperture of 2D-bar code type, that the general shape of the 2D-PSF shape changes depending on the value of the distance d, which can be useful to boost even more the algorithm speed. The 2D-bar coded aperture enables to detect unambiguously the direction into which the optical power of the active lens(es) should move and thus enables a quicker adjustment of the optical power of the vision compensating device 110, 120 to target faster the user's prescription. The 2D-bar coded aperture is useful especially in the case of Figure 6 and 7, when the level of blur of an image of the retina (and not the PSF size) is the criterion used to approximate the refractive error.

Of course, the asymmetrical aperture or the 2D-bar coded aperture can be used for each pupil 36, 46 of a binocular apparatus according to the present disclosure.

It is to be noted that for the evaluation of the refraction error, it is not mandatory to measure the pupil size, because the refraction error can be measured by minimizing the spot size. However, knowing the pupil size enables to accelerate the measurement of the refraction error.

Figure 6 schematically represents an apparatus for determining refraction error of an eye of a subject according to a second embodiment. Only the differences with the first embodiment will be detailed.

In the second embodiment (see figure 6), the apparatus further comprises a beam splitter 15, 25 placed between the eye 1, 2 of the user and the vision compensating device 110, 120. Advantageously, the beam splitter 15, 25 comprises a hot mirror adapted for reflecting near infrared light from the light source 31, 41 and for transmitting light in the visible range. The light source 31, 41 and the illumination optical system 33, 43 form the incident light beam 32, 42 which is focused in the vicinity of the retina of the eye 1, 2 of the user. In the second embodiment, the incident light beam 32, 42 do(es) not pass through the vision compensating device 110, 120 nor through the pupil 36, 46. The incident light beam 32, 42 is reflected on the retina of the eye 1, 2 and forms a reflected light beam 34, 44. The reflected light beam 34, 44 passes through the crystalline lens 3, 4 of the eye 1, 2, through the beam splitter 15, 25 and then through the vision compensating device 110, 120. The beam-splitter 10, 20 directs at least a part of the reflected light beam 34, 44 towards the measurement system of the refraction error 310, 320. The illumination optical system 33, 43 collects the reflected light beam 34, 44 passing through the pupil 36, 46 and directs the reflected light beam 34, 44 towards the image detection system 35, 45.

The second embodiment enables direct illumination of the retina. In this case, the incident light beam 32, 42 coming from the LED light source 31, 41 is collimated and does not cross the active optical module 110, 210. The optical module parameters are adjusted until the image of the retina detected by the detection system 35, 45 is the sharpest. An advantage of the second embodiment is that the shape of the light pattern on the retina gives clues on the user's refraction needs, that can be used to accelerate the image processing algorithm.

In the second embodiment, a spot on the retina is observed. However, the aim of the refraction measurement is no longer to reduce the size of this spot, but to sharpen the image of the retina. Thus, it is particularly useful to use a pupil 36, 46 with a coded aperture as described in relation with figure 4, on the imaging optical system in order to estimate faster the user refraction needs.

In the third embodiment (see figure 7), the apparatus also comprises a beam splitter 15, 25 placed between the eye 1, 2 of the user and the vision compensating device 110, 120. However, the light source 31, 41 and the illumination optical system 33, 43 form the incident light beam 32, 42 which is focused in the vicinity of the eye cornea of the user. In the third embodiment, the incident light beam 32, 42 does not pass through the vision compensating device 110, 120. The incident light beam 32, 42 illuminates a large area of the retina of the eye 1, 2 and is reflected on the retina of the eye 1, 2 thus forming the reflected light beam 34, 44. The reflected light beam 34, 44 passes through the crystalline lens 3, 4 of the eye 1, 2, through the beam splitter 15, 25 and then through the vision compensating device 110, 120. The beam-splitter 10, 20 directs at least a part of the reflected light beam 34, 44 towards the measurement system of the refraction error 310, 320. The illumination optical system 33, 43 collects the reflected light beam 34, 44 passing through the pupil 36, 46 and directs the reflected light beam 34, 44 towards the image detection system 35, 45.

The third embodiment enables direct observation of a wide light beam on the retina, see Figure 7. In this case, the incident light beam 32, 42 coming from the LED light source 31, 41 is focused in the vicinity of the eye cornea and does not cross the active optical module. The optical module parameters are adjusted until the image on the retina is the sharpest. The advantage of the third embodiment is that the illuminated zone on the retina is wider, which is useful for the algorithm, and which enables to detect abnormalities on the retina.

Advantageously, the apparatus comprises a remote controller adapted for driving the device for varying the variable power of said optical system. The remote controller comprises for example a tablet 50 or a smartphone or a computer adapted for wireless communication such as Bluetooth connection with the apparatus for determining refraction error of at least an eye of a subject according to the present disclosure (see figure 8).

Alternatively, the apparatus for determining refraction error of at least an eye of a subject comprising digital trial lens is directly connected to the internet via a Wi-Fi connection and remotely operated by an eye care practitioner (ECP), optometrist or eye doctor.

The apparatus according to the present disclosure offers a mobile system combining objective and subjective refraction, with the advantages of both methods. The objective refraction measurement is quick and automatic. This combined and integrated apparatus enables to determine refraction in a more robust way while enabling to manage accommodation. The apparatus according to the present disclosure is advantageous in terms of costs compared to two separate apparatuses, one for objective refraction and another for subjective refraction.

### Process

An example of combined refraction process in far vision is as follows:
a) placing a variable optical power corrective system 111, 121 mounted in an eyewear device 100 adapted to be worn by the user so that the user is able to see a target scene through the variable power optical system;
b) emitting a light beam 32, 42 and directing the light beam 32, 42 towards the eye 1, 2 of the subject so as to form a reflected light beam 34, 44 by reflection on the retina of the eye 1, 2;
c) directing the reflected light beam 34, 44 towards an image detection system 35, 45 by transmission through the variable power optical system 111, 121 and by reflection on a beam splitter 10, 20, the variable power optical system 111, 121 being arranged between the eye 1, 2 of the subject and the beam splitter 10, 20, the beam splitter 10, 20 enabling simultaneously the user to see the target scene through the optical beam splitter 10, 20 and the variable power optical system 111, 121;
d) detecting an image of the reflected light beam 34, 44 formed on the image detection system 35, 45;
e) processing said image to deduce therefrom as estimation of the refraction error of said eye 1, 2 and a feedback signal depending on the estimation,
f) applying the feedback signal for varying the variable power of said optical system 111, 121;
g) iterating the steps d) to f) until an image of the target scene on the retina meets a predetermined ending criterion.

Preferably, the ending criterion relates to an evaluation for the size of said image of the target scene on the retina, said size being the minimum size in at least one direction.

According to a particular aspect, after step a) and before step b), the optical power of the vision compensating device 110, respectively 120 is set to zero (0D).

According to a particular aspect, after step a) and before step b), a spherical power (for example 2D)is added to the vision compensating device 110 to relax the accommodation.

Advantageously, an estimation of the refraction error (Sph/Cyl/Axis) for both eyes is performed using the spread point on the image detection system 35, 45.

Advantageously, the method further includes a step of checking a visual acuity of said eye based on a feedback signal of the wearer viewing the target scene through the corrective system and the beam splitter and eventually adjusting the variable power to increase the visual acuity of said eye if the value of visual acuity is under a determined threshold (for example 10/10), based on the feedback of the subject. This step is iterated until the visual acuity value is over the threshold.

After the step g), the optical power of the vision compensating device 110, respectively 120 is set to compensate the refraction error or a part of the refraction error of each eye, and a subjective refraction measurement is performed.

The optical power of the vision compensating device 110, respectively 120 is finely adjusted a few times until reaching a suitable correction of the vision compensating device 110, respectively 120 for the eye 1, respectively 2 of the wearer.

The combined refraction process can be performed in monocular or binocular vision conditions, and at various vision distances such a far vision, near vision, or intermediate vision.

## Claims

1. Apparatus for determining refraction error of at least an eye of a subject, the apparatus comprising:
- a corrective system comprising a variable power optical system mounted in an eyewear device adapted to be worn by the user so that the user is able to see a target scene through the variable power optical system and a device for varying the variable power of said optical system;
- a beam splitter, the variable power optical system being arranged between the eye of the subject and the beam splitter,
- a measurement system of the refraction error of said eye, the measurement system comprising a light source adapted to emit a light beam, an illumination optical system adapted to direct the light beam towards the eye of the subject so as to form a reflected light beam by reflection on the retina of the eye, and an image detection system;
- the beam splitter being adapted to direct the reflected light beam towards the image detection system, the beam splitter enabling simultaneously the user to see the target scene through the optical beam splitter and the variable power optical system;
- the image detection system being adapted to detect an image of the reflected light beam by transmission through the variable power optical system;
- a computing system comprising an image processing system adapted to process said image and deduce therefrom an estimation of the refraction error of said eye, the computing system being adapted to send a feedback signal to the device for varying the variable power of said optical system, the feedback signal depending on the estimation of the refraction error of said eye.

2. Apparatus according to claim 1 wherein the image processing system is adapted to determine a two-dimension point spread function of the image of the reflected light beam and to deduce the estimation of the refraction error of said eye from the two-dimension point spread function.

3. Apparatus according to claim 2 wherein the measurement system comprises a coded aperture, said coded aperture having an asymmetrical shape with respect to a rotation about an optical axis of said measurement system.

4. Apparatus according to claim 3 wherein the coded aperture comprises a portion of a disk or a two-dimensional bar code.

5. Apparatus according to claim 1 wherein the image processing system is adapted to determine a sharpness of the image of the reflected light beam and to deduce the estimation of the refraction error of said eye from the sharpness.

6. Apparatus according to any one of claim 1 to 5 wherein said apparatus is adapted to enable determination of a visual acuity of the eye viewing the target scene through the corrective system and the beam splitter.

7. Apparatus according to any one of claim 1 to 4 and 6 comprising another beam splitter arranged between the variable power optical system and the eye of the subject.

8. Apparatus according to any one of claim 1 to 7 wherein the illumination optical system is adapted to collimate the light beam on a cornea of the eye or, respectively, focus the light beam on the cornea of the eye.

9. Apparatus according to any one of claim 1 to 8 comprising means for moving the target scene and wherein the measurement system is adapted to estimate an accommodation of the eye in response to the moving of the target scene.

10. Apparatus according to any one of claim 1 to 9 wherein the apparatus is adapted for binocular vision.

11. Apparatus according to claim 10 comprising an adjustment system for adjusting an interpupillary distance.

12. Method for determining refraction error of at least an eye of a subject, the method comprising the steps of:
a) placing a variable power optical system mounted in an eyewear device adapted to be worn by the user so that the user is able to see a target scene through the variable power optical system;
b) emitting a light beam and directing the light beam towards the eye of the subject so as to form a reflected light beam by reflection on the retina of the eye;
c) directing the reflected light beam towards an image detection system by transmission through the variable power optical system and by reflection on a beam splitter, the variable power optical system being arranged between the eye of the subject and the beam splitter, the beam splitter enabling simultaneously the user to see the target scene through the optical beam splitter and the variable power optical system;
d) detecting an image of the reflected light beam formed on the image detection system;
e) processing said image to deduce therefrom an estimation of the refraction error of said eye and a feedback signal depending on the estimation,
f) applying the feedback signal for varying the variable power of said optical system;
g) iterating the steps d) to f) until an image of the target scene on the retina meets a predetermined ending criterion.

13. Method according to claim 12 further comprising a step of checking a visual performance of said eye based on a feedback signal of the wearer viewing the target scene through the corrective system and the beam splitter.
